# EUROPÄISCHE PATENTANMELDUNG

(11) **EP 4 847 624 A1**
(43) Veröffentlichungstag der Anmeldung: **02.12.2026**
(21) Anmeldenummer: 25180036.3
(22) Anmeldetag: 30.05.2025
(51) Int. Cl.: C12P 19/36, C12N 9/04, C12N 9/02, C12P 17/04

(54) **HERSTELLUNG VON 2,5-FURANDICARBONSÄUREALKYLESTER**

(71) Anmelder: Annikki GmbH, 8074 Raaba-Grambach (AT)
(72) Erfinder: Staunig, Nicole, 8074 Raaba-Grambach (AT)
(74) Vertreter: Schwarz & Partner Patentanwälte GmbH

(57) **Zusammenfassung**

Verfahren zur Herstellung eines 2,5-Furandicarbonsäurealkylesters der allgemeinen Formel (I) 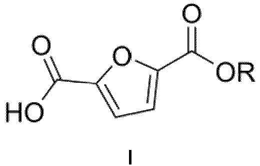 in der R für C1-C4-Alkyl steht,
indem ein 5-Formyl-2-furancarbonsäurealkylester der allgemeinen Formel (II) 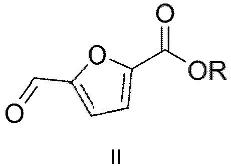 in der R für C1-C4-Alkyl steht,
und welcher in einer wässrigen Lösung vorliegt, durch Behandeln mit einer ersten NAD(P)⁺-abhängigen Oxidoreduktase oxidiert wird, wobei das bei der Oxidation entstandene NAD(P)H enzymatisch mittels einer zweiten Oxidoreduktase wieder zu NAD(P)⁺ oxidiert wird, wonach der 2,5-Furandicarbonsäurealkylester der allgemeinen Formel (I) abgetrennt wird.

## Beschreibung

Die Erfindung betrifft ein Verfahren zur Herstellung eines 2,5-Furandicarbonsäurealkylesters der allgemeinen Formel (I) 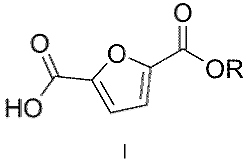 in der R für C1-C4-Alkyl steht.

### Hintergrund der Erfindung

Im Jahr 2018 basierten in etwa 90% der produzierten Kunststoffe weltweit (400 Mt) auf fossilen Rohstoffen, die restlichen Anteile entfielen auf recycelte (9%), biobasierte (1%) und CO₂-basierte Kunststoffe (<1%) (Carus et al., 2020). Gleichzeitig wächst auch der Bedarf an Kunststoffen weltweit. 2019 lag die jährliche CO₂-Emission des gesamten Lebenszyklus von Kunststoffen bei 0,86 Gt, was einer CO₂-Emission von 189 Kohlekraftwerken, die mit voller Leistung (500 MW) laufen, entspricht. Für 2050 wird ein Anstieg auf 2,8 Gt (entspricht 615 Kohlenkraftwerken) prognostiziert (Hamilton et al., 2019).

Einer der Kunststoffe, die auf fossilen Rohstoffen basieren, ist Polyethylenterephthalat (PET), das vor allem in Form von Getränkeverpackungen Anwendung findet. Es handelt sich dabei ein Kondensationspolymer, welches aus Terephthalsäure (1,4-Benzoldicarbonsäure) und Ethylenglycol (Ethan-1,2-diol) unter Wasserabspaltung hergestellt wird.

Terephthalsäure wird großtechnisch durch Oxidation von *p*-Xylol (1,4-Dimethylbenzol) mit Luftsauerstoff bei ca. 200 °C in Gegenwart von Kobalt-Acetat, Mangan-Acetat und HBr in Essigsäure im sogenannten AMOCO-Prozess hergestellt (Tomäs et al., 2013).

Ethylenglycol wird durch die Hydrolyse von Ethylenoxid (bei 200 °C), welches wiederum durch die Oxidation von Ethen (gewonnen aus fossilen Rohstoffen) erhalten wird, hergestellt (Berger, 2016). Aufgrund der fossilen Quellen der Ausgangsstoffe (p-Xylol und Ethen) kann PET im Allgemeinen nicht als nachhaltig bezeichnet werden.

Um das 1,5-Grad-Ziel des Pariser Klimaabkommens zu erreichen und somit die negativen Folgen des Klimawandels zu begrenzen, müssen nachhaltige Alternativen für die petrochemisch basierten Kunststoffe gefunden werden. Für Ethylenglycol gibt es beispielsweise Verfahren, die auf erneuerbaren Ressourcen aufbauen. So kann beispielsweise Ethen auch durch die Dehydratisierung von Bio-Ethanol, der fermentativ aus Glucose oder Stärke erhalten wurde, hergestellt werden (z.B. Fan et al., 2013). Auch fermentative Umsetzungen von Xylose und/oder Glucose zu Ethylenglycol mittels metabolisch engineerten Mikroorganismen (Escherichia coli oder Saccharomyces cerevisiae) sind bekannt (Salusjärvi et al., 2019).

Der zweite Baustein von PET, die aromatische Verbindung Terephthalsäure, kann schwer aus nachhaltigen Rohstoffen hergestellt werden und muss daher ersetzt werden. Eine exzellente Substitution für die Terephthalsäure ist die 2,5-Furandicarbonsäure (FDCA), welche überwiegend aus dem katalytischen Upgrading von Biomasse erhalten wird. Durch die Polymerisation von FDCA mit Ethylenglycol entsteht PEF (Polyethylenfuranoat), das in der Struktur anstelle des Benzol-Rings einen heteroaromatischen Furan-Ring trägt. Wie PET ist PEF ein Thermoplast, zeichnet sich aber im Vergleich zu PET durch eine signifikant höhere Bioabbaubarkeit und bessere thermische (höhere Glasübergangstemperatur, niedrigere Schmelztemperatur) sowie mechanische Eigenschaften (höhere Steifheit) aus. Die wichtigste Eigenschaft von PEF aber ist die verringerte Permeabilität für Gase wie O₂ und CO₂. Dies ist besonders für Getränke wichtig, da dadurch die Haltbarkeit von Getränken (Verhinderung von Ausgasen karbonisierter Getränke; Verhinderung von Oxidationsprozessen durch diffundierten Sauerstoff) verlängert werden kann (de Jong et al., 2022).

Zur Herstellung von PEF kann FDCA aber auch in Form von Estern wie dem Dimethylester (FDCM) eingesetzt werden, welcher durch Zugabe von Ethylenglycol umgeestert wird - das freiwerdende Methanol wird aus der Reaktionsmischung entfernt, um das Gleichgewicht Richtung FDCA zu verschieben (Yu et al., 2016).

Zur Herstellung von FDCA-Estern sind bereits einige Verfahren bekannt.

Die US 8519167 B2 beschreibt die Oxidation von 5-Hydroxymethylfurfural (HMF), 5-Alkoxymethylfurfural oder 2,5-Di(alkoxymethyl)furan zu FDCA oder dessen Alkylester mit Sauerstoff als Oxidationsmittel, wobei als Katalysator eine Mischung aus Cobalt-Acetat, Mangan-Acetat und Natriumbromid gelöst in Essigsäure verwendet wurde. Die Ausbeuten für die Reaktionen lagen bei bis 77 %, wobei bei Verwendung von 5-Alkoxymethylfurfural als Substrat eine Mischung aus FDCA und dem entsprechenden Monoester (Methyl oder Ethyl) erhalten wurde.

Salazar et al. (2020) beschreiben die oxidative Veresterung von HMF zu FDCM mit O₂ und einem heterogenen bimetallischen Co/Ru-Katalysator auf einem N-gedopten Kohlenstoff-Support in Methanol. Neben dem gewünschten Diester werden auch Nebenprodukte wie 2-((5-(Methoxycarbonyl)furan-2-yl)methyl)-5-methyl-furan-2,5-dicarboxylat in signifikanten Mengen (bis zu 20%) gebildet.

Chen et al. (2023) beschreiben die chemoenzymatische Herstellung von FDCA-Estern ausgehend von D-Gluconat, welches im ersten Schritt enzymatisch zu 5-Keto-Gluconat oxidiert wird. Durch chemische Zyklisierung mit Schwefelsäure in Gegenwart von Methanol oder n-Butanol entstehen die entsprechenden FFA-Ester. Der n-Butylester von FFA wurde in weiterer Folge chemisch mittels Pt/C-Katalysator und O₂ zum FDCA-n-Butylester oxidiert. Alternativ kann der letzte Oxidationsschritt auch mit KMnO₄ und O₂ bewerkstelligt werden (siehe CN 107954959 A).

Die chemischen Oxidationsverfahren können aufgrund der verwendeten Chemikalien wie Übergangsmetall-Verbindungen oder der harschen Reaktionsbedingungen nicht als nachhaltig bezeichnet werden. Darüber hinaus treten je nach Verfahren Nebenprodukte in signifikanten Mengen auf.

Hier setzt nun die folgende Erfindung an und möchte ein Verfahren zur Herstellung von 2,5-Furandicarbonsäurealkylester zur Verfügung stellen, welches die oben genannten Nachteile nicht aufweist.

### Detaillierte Beschreibung der Erfindung

Die Aufgabe wird mit einem Verfahren zur Herstellung eines 2,5-Furandicarbonsäurealkylesters der allgemeinen Formel (I) 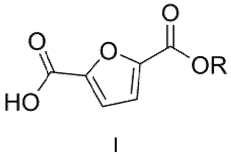 in der R für C1-C4-Alkyl steht, erfindungsgemäß gelöst, indem ein 5-Formyl-2-furancarbonsäurealkylester der allgemeinen Formel (II) 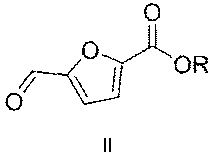 in der R für C1-C4-Alkyl steht, und welcher in einer wässrigen Lösung vorliegt, durch Behandeln mit einer ersten NAD(P)⁺-abhängigen Oxidoreduktase oxidiert wird, wobei das bei der Oxidation entstandene NAD(P)H enzymatisch mittels einer zweiten Oxidoreduktase wieder zu NAD(P)⁺ oxidiert wird, wonach der 2,5-Furandicarbonsäurealkylester der allgemeinen Formel (I) abgetrennt wird.

C1-C4 steht für Methyl, Ethyl, *n*-Propyl, *iso*-Propyl, *n*-Butyl, *iso*-Butyl, *sec*-Butyl oder *tert*-Butyl.

Die Verbindungen der allgemeinen Formeln (I) und (II) stellen somit die Monoalkylester dar.

Der 5-Formyl-2-furancarbonsäurealkylester der allgemeinen Formel (II) kann in der wässrigen Lösung als Feststoff, in flüssiger Form oder in der wässrigen Lösung gelöst vorliegen.

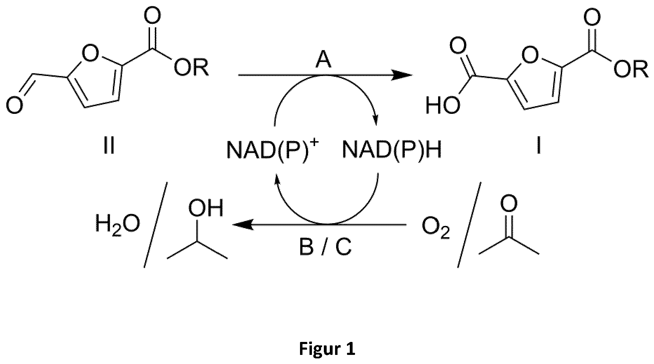

Das erfindungsgemäße Verfahren ist in Figur 1 schematisch dargestellt. Die Bezeichnung I steht für 2,5-Furandicarbonsäurealkylester, II für 5-Formyl-2-furancarbonsäurealkylester, A für Aldehyd-Dehydrogenase, B für NAD(P)H-Oxidase und C für Alkoholdehydrogenase.

Es hat sich überraschenderweise gezeigt, dass 5-Formyl-2-furancarbonsäurealkylester ohne die oben genannten Nachteile biokatalytisch zu den entsprechenden 2,5-Furandicarbonsäurealkylesters oxidiert werden können.

Besonders bevorzugt ist der 5-Formyl-2-furancarbonsäurealkylester ein Methylester (R = CH₃).

Bei der ersten Oxidoreduktase handelt es sich bevorzugt um eine Aldehyd-Dehydrogenase.

Bei der zweiten Oxidoreduktase handelt es sich bevorzugt um eine Dehydrogenase, besonders bevorzugt um eine Alkoholdehydrogenase, oder eine NAD(P)H-Oxidase.

Besonders bevorzugt umfasst die Aldehyd-Dehydrogenase eine Aminosäuresequenz oder besteht aus dieser, welche ausgewählt ist aus der Gruppe bestehend aus:
i) einer Aminosäuresequenz, die eine Identität zu SEQ ID Nr. 2, SEQ ID Nr. 4, SEQ ID Nr. 6 oder SEQ ID Nr. 8 von mindestens 80% aufweist,
ii) einer Aminosäuresequenz, die durch eine Nukleinsäure kodiert wird, die eine Identität zu SEQ ID Nr. 1, SEQ ID Nr. 3, SEQ ID Nr. 5 oder SEQ ID Nr. 7 von mindestens 80% aufweist, und
iii) einer Aminosäuresequenz, die durch eine Nukleinsäure kodiert wird, die unter stringenten Bedingungen an einen komplementären Strang eines ein Nukleinsäuremoleküls mit der Nukleinsäuresequenz SEQ ID Nr. 1, SEQ ID Nr. 3, SEQ ID Nr. 5 oder SEQ ID Nr. 7 bindet.

SEQ ID Nr. 1: 
ATGTCACGCTATGAACTGCTGATCGATGGTCGCCTGCAGGCGGCCGAGCACTACGACCGGGTGATCGA
CCCGGCCAGCGAAGAAATCGTTGGCGAAGCCGCCCGCGCCAGCCTGGAGCAGGTCGACGCGGCGGTG
GATGCCGCACACCGCGCCTTCCCGGCCTGGGCCACCGATCTCGACGTCCGCCGCCAGAGCCTGGCCCG
AGCCGCCGAACGAGTACGCGAGAATGCCCAGGCGCTGGCCGAGCTGATCACCCGCGAACAGGGTCGC
CCACTGCGCTCGACCCTGGAGGAAGTGGCTGGCGTCGCCGCCACTTTCGAGCACCACGCGCAGCTGGA
 
GCTACCCGCCGACACCCAGTTGCGCGACGACGGCGAGCGCCTGGTGCGCATCACCCGCAAACCGCTGG
GGGTGGTCGCCGCGATCACCCCGTGGAACGTCCCGCTGATCCTGCTGGTATTGAAGATCGCGCCTGCC
CTGCACGCCGGCAACACCGTGGTGGCCAAGCCCTCGGAGCACACGCCGCTGTCTACCCTGCTGCTGGC
GCGACTGCTGGGCGATGTGTTCCCCGCTGGCGTGTTCAACGTGGTTGCCGGTGCGGGCGAGGTTGGC
GAACACCTGGTACGTCATCCGCGGGTGCGCCACGTGACCTTCACCGGCAGCGTCGCCACCGGCAAGCG
CCTGTATGCCGGCGCGGGGGACGACCTCAAGCGTCTTACCCTGGAACTGGGCGGCAACGACGCCGCG
CTGGTACTGGAAGACGCCGACCTCGACGCCATCGTCGAACCGCTGTTCTGGGGCGCCTTCTGGAACAG
CGGCCAGGTGTGCTTTGCGATCAAGCGCCTGTACGTGCATGACAGCCTGTTCGAACCGTTACTGGCGA
AACTCGCCGAGCGCGCCCAGCGCACCCGCCTCGGTCATGGGCTCGACCCGCAGACGGAACTGGGGCC
GCTGACCAACGCACAGCAACTGGAACGGGTCATCGCCCTGGTGGAAGACGCCAAGGCTCACGGAGCA
CGCATCCGCAGCGGCGGCGTACGGCCCGACGGTCCCGGCTACTTCTACCCGCCGACCCTGGTCAGCGG
CGTGGCGGCCGGCGTCGCGCTGGTGGACGAGGAACAGTTCGGCCCGGTGTTGCCGGTGATCTCCTTCC
GCAACGAAGAGGACGCCATTACCCAGGCCAACGCCAGCCACTACGGCCTCGGCGCCTCGGTGTGGACC
CGCGACCTGGCGCGCGGCGAAGCCATCGCCAGGCGGCTGGAGGCGGGCCTGGCCTGGGTCAACCAGC
ACGGCCACATCCAGCCCGGCGCGCCCAAGGGCGGGCACAAGTGGAGCGGGCTCGGCTACGAAGGCG
GGCAGCGCGGCTATGAGGCCTTCAGCGAGCTGCAGGTGCTGAACATTTCGCGGCGCTAA
SEQ ID Nr. 2: 
MSRYELLIDGRLQAAEHYDRVIDPASEEIVGEAARASLEQVDAAVDAAHRAFPAWATDLDVRRQSLARAAE
RVRENAQALAELITREQGRPLRSTLEEVAGVAATFEHHAQLELPADTQLRDDGERLVRITRKPLGVVAAITP
WNVPLILLVLKIAPALHAGNTVVAKPSEHTPLSTLLLARLLGDVFPAGVFNVVAGAGEVGEHLVRHPRVRHV
TFTGSVATGKRLYAGAGDDLKRLTLELGGNDAALVLEDADLDAIVEPLFWGAFWNSGQVCFAIKRLYVHDS
LFEPLLAKLAERAQRTRLGHGLDPQTELGPLTNAQQLERVIALVEDAKAHGARIRSGGVRPDGPGYFYPPTL
VSGVAAGVALVDEEQFGPVLPVISFRNEEDAITQANASHYGLGASVWTRDLARGEAIARRLEAGLAWVNQ
HGHIQPGAPKGGHKWSGLGYEGGQRGYEAFSELQVLNISRR
SEQ ID Nr. 3: 
ATGTCGACATTTCATTTACTTATTGATGGCCATCTGCAAGCCAGTGACCAATCCGATGTGGTGATCAACC
CCGCGACCGAGCTGGAAGTCGGGCGTGCCCCACGTGCCAGCGCCACCCAGGTCGACCAGGCAGTGGA
AGCCGCACATCAGGCATTTCATCGCTGGGCATCACAGCCTGAGGTGCGCCAGCAGGCACTGCTGGGCG
CGGCCGCCGCCATTCGCCAGCATGCCGATGCCCTGGCACGCCTGATCACGCAAGAGCAGGGGCGGCCA
TTGCACTTTACCCAGGGCGAGGTGGCCGGGGCTGCTGCTACCTTTGAACACTATGCCGGGTTTGCCGCG
CCATCGGATGTGGTGCTGCAGCAGGACGAACAAAGCGGGTCAGCATTGAGCGCAGGCCGTTTGGCG
TAGTGGCTGCCATTACGCCCTGGAATGTGCCCATCATCCTGCTGGTACTGAAAATCGCCCCGGCCTTGA
 
AGGCTGGCAATACCGTGGTCGCCAAGCCATCGGAATACACCCCGCTTTCTACCTTGTACCTGGGTGAAA
TTCTGAAAGATGTATTTCCGCCCGGCGTGCTGAACGTGATAGCCGGTGACGGTCAGGTGGGGGCGCGC
CTGGCATCGCATCCGCTGGTGCAGAAGGTGACGTTCACCGGCAGCGTGGCAACCGGAAAAAAACTCTA
TGCCAGCGCCGCGCAGGATGTAAAACGCCTGACGCTGGAACTGGGCGGTAACGATGCGGCCATCGTG
CTGGACGATGCCAATGTCGATGCCATTGCCGAGAAGATTTTCTGGGGCGCCTTCTGGAATAGCGGTCA
GGTGTGCTTTGCCATCAAGCGCCTCTATGTGCACGAGCGTGTTTTCCAGCCCTTGCTCGATGCCCTGGT
GAAACGCGCGCAAAAAACCCGCGTGGGCGATGGCCAGCTGCCGGGTACCGAGCTGGGGCCGCTTACC
AACAAAGCCCAGTTTGAGCGCGTGATATCGCTGGTGGAAGACGCCAGACGCCATGGCGCCACCATTCA
TTCAGGCGGTGCTGCATTGCCTGGCCCCGGCTATTTCTATCCGCCCACCCTGGTCACCGGCATAGGCGC
AGGTGTCGCGCTGGTGGATGAAGAGCAGTTCGGACCAGTATTGCCGCTGATTCCTTTCCGTGATGAGC
AAGAGGCGGTGCGTCAGGCCAACGACAGTCCGTTTGGTCTGGGCGCTTCGGTTTGGACCGCCAATCCC
GAACGCGGCCTGGCGCTGGTACGCCAGCTCCAGGCCGGGCTCGCCTGGGTCAACCAGCATGGCGATAT
CCATCCCGGTGCGCCCAAGGGCGGCTACAAATCCAGTGGCCTTGGCTACGAGGGCGGGCTGCGTGGCT
ATGACGAGTTCAGCGAGCTGCAAGTCGTCAATGCGGCGCTGGTTTAA
SEQ ID Nr. 4: 
MSTFHLLIDGHLQASDQSDVVINPATELEVGRAPRASATQVDQAVEAAHQAFHRWASQPEVRQQALLGA
AAAIRQHADALARLITQEQGRPLHFTQGEVAGAAATFEHYAGFAAPSDVVLQQDEQKRVSIERRPFGVVAA
ITPWNVPIILLVLKIAPALKAGNTVVAKPSEYTPLSTLYLGEILKDVFPPGVLNVIAGDGQVGARLASHPLVQK
VTFTGSVATGKKLYASAAQDVKRLTLELGGNDAAIVLDDANVDAIAEKIFWGAFWNSGQVCFAIKRLYVHE
RVFQPLLDALVKRAQKTRVGDGQLPGTELGPLTNKAQFERVISLVEDARRHGATIHSGGAALPGPGYFYPPT
LVTGIGAGVALVDEEQFGPVLPLIPFRDEQEAVRQANDSPFGLGASVWTANPERGLALVRQLQAGLAWVN
QHGDIHPGAPKGGYKSSGLGYEGGLRGYDEFSELQVVNAALV
SEQ ID Nr. 5: 
ATGAGCGATTCCCGTTATACCGACCTCGGTCTCCAGCCCCTGGCCGGCGAGTGGCGCCACGGCCGGGC
CGGCCGCCGGCTGAAGGTGAGCAACCCGTTCGACGGCAGCCTGCTGCTGGAGATCGAGCAGGCCGAC
CGCGACGACCTCGATGCCGCCTACGCCAAGGCCGCCGAGGTCCAGCCGGCATGGGCCGCGCTCGGGC
CCTCGGCACGCGCGGCGGTACTGTACAAGGCGGTGGAGGTGTTCGACCGCCGCCACGAGGAGATCGT
CGACTGGATCATCCGCGAGTCCGGCAGCACCCGCCTGAAGGCCGAGATCGAATGGGGCGCGGCGCGC
GCGATCACCCTGGAGTCGGCGTCGTTCCCGGCACGGGTGCACGGCGCATCGTCGAGTCCGACGTGCC
GGGCAAGGAAAGCCGGGTCTACCGCAGCGCCATCGGCGTGGTCGGGGTGATCAGCCCGTGGAACTTC
CCGCTGCACCTGACCCAGCGTTCCATCGCCCCGGCCCTGGCGCTGGGCAACGCGGTGGTGGTCAAGCC
GGCCAGCGACACGCCGGTCTGCGGCGGACTGCTGCTGGCGCGGATCTTCGAAGAGGCCGGGCTGCCG
 
GCCGGGCTGTTCAGCGTGGTGGTCGGCCCCGGCAGCGAGATCGGCGACGCCTTCGTCGAGCACCCGG
TGCCGGGCCTGGTGACCTTCACCGGATCGACCCCGGTGGGCCGCAACATCGGCCGCATCGCCAGCGGC
GGCGCGCACCTCAAGCACGTGGCGCTGGAGCTGGGCGGCAACAGTCCGTTCGTGGTGCTCGGCGACG
CCGATCTGGAGCAGGCGGTGAATGCCGCGGTGTTCGGCAAGTTCCTCCACCAGGGGCAGATCTGCATG
GCGATCAACCGCATCATCGTCGAGGACAGCCTCTACGACGCTTTCGCCGCGCGCTTCGTCGAGCGGGTC
AAGGGTCTCCGGGTCGGCGATCCGCAGCGCGCCGATACCGCGGTCGGGCCGATCGTCAACGCGCGCC
AGCTCGAAGGCCTGCTGGAAAAGATCCGCCTGGCCCGCCAGGAAGGCGCCAAGCCGCTGTACGAGGG
CGGCGTCGATGGGCAGTTGCTGGCTCCGCACGTATTCGGCGAGGTCACCGCGACGATGGAGATCGCCC
GCGATGAAATCTTCGGCCCGCTGGTCGGCCTGCTCCGCGCGCGACGAGGCGCATGCGCTGGAGTTG
GCCAACGCCAGCGAATACGGGCTGTCCAGCGCGGTGTTCAGCCGCGACCTGGAACGCGCGGTGCGCTT
TGCCCGCCAGCTTCGCGCGGGGATGACCCACGTCAACGACATTCCGGTGAACGACGAGGCCAACGCGC
CCTTCGGCGGCGAGAAGAACTCCGGACTTGGCCGCTTCAACGGCGACTGGGCCATCGAGGAATTCACC
ACCGACCACTGGATCAGCGTGCAGCACGCGCCGCGCCAGTACCCGTTCTGA
SEQ ID Nr. 6: 
MSDSRYTDLGLQPLAGEWRHGRAGRRLKVSNPFDGSLLLEIEQADRDDLDAAYAKAAEVQPAWAALGPSA
RAAVLYKAVEVFDRRHEEIVDWIIRESGSTRLKAEIEWGAARAITLESASFPARVHGRIVESDVPGKESRVYRS
AIGVVGVISPWNFPLHLTQRSIAPALALGNAVVVKPASDTPVCGGLLLARIFEEAGLPAGLFSVVVGPGSEIG
DAFVEHPVPGLVTFTGSTPVGRNIGRIASGGAHLKHVALELGGNSPFVVLGDADLEQAVNAAVFGKFLHQG
QICMAINRIIVEDSLYDAFAARFVERVKGLRVGDPQRADTAVGPIVNARQLEGLLEKIRLARQEGAKPLYEGG
VDGQLLAPHVFGEVTATMEIARDEIFGPLVGLLRARDEAHALELANASEYGLSSAVFSRDLERAVRFARQLR
AGMTHVNDIPVNDEANAPFGGEKNSGLGRFNGDWAIEEFTTDHWISVQHAPRQYPF
SEQ ID Nr. 7: 
ATGAAATCGTATCAGGGATTGGCTGACAAGTGGATTAAGGGCAGTGGGGAAGAATACCTTGATATTAA
TCCGGCTGATAAGGATCACGTATTAGCTAAGATAAGATTATATACAAAAGATGACGTTAAAGAAGCTAT
AAACAAGGCTGTAGCCAAATTCGACGAATGGTCAAGGACTCCAGCACCTAAAAGAGGCTCAATATTAC
TTAAGGCAGGGGAATTAATGGAACAAGAAGCCCAAGAGTTTGCGCTATTGATGACATTAGAGGAGGG
TAAGACTCTCAAGGATAGTATGTTTGAAGTGACAAGAAGTTATAATTTACTGAAATTTTATGGAGCATT
AGCATTTAAGATATCTGGGAAAACGCTTCCTTCAGCAGATCCTAATACTAGGATATTTACAGTAAAGGA
ACCCTTAGGCGTAGTAGCTTTAATTACGCCGTGGAATTTCCCATTATCAATACCAGTATGGAAATTGGCT
CCAGCCTTGGCTGCGGGTAACACTGCAGTAATAAAACCAGCGACGAAAACACCGTTAATGGTAGCCAA
ATTGGTAGAAGTGTTGTCTAAAGCTGGATTGCCAGAGGGTGTCGTGAATTTAGTAGTTGGTAAGGGAA
GTGAAGTCGGAGATACCATAGTAAGTGATGATAATATAGCTGCAGTATCATTTACTGGATCAACCGAG
 
GTAGGTAAGAGAATTTACAAACTCGTAGGAAATAAAAATAGAATGACAAGAATTCAACTAGAGCTAGG
AGGTAAAAACGCGTTATATGTGGATAAGAGCGCTGACTTAACGTTAGCTGCTGAATTAGCCGTAAGAG
GAGGATTTGGACTAACCGGTCAATCATGTACTGCAACTAGTAGGTTAATAATTAACAAGGATGTATATA
CTCAATTTAAACAAAGACTACTAGAAAGAGTTAAGAAGTGGAGAGTAGGACCGGGTACTGAAGATGTT
GATATGGGTCCAGTTGTAGATGAAGGTCAATTTAAGAAAGACTTAGAATATATAGAATACGGAAAGAA
TGTGGGAGCAAAACTAATTTATGGTGGAAATATAATACCAGGGAAGGATATTTCCTAGAACCTACAA
TTTTCGAAGGAGTCACATCTGATATGAGGCTATTTAAAGAAGAGATTTTCGGTCCAGTACTTAGTGTCA
CTGAGGCAAAAGATTTAGATGAGGCTATAAGGCTAGTTAACGCTGTAGACTATGGACATACAGCTGGA
ATAGTCGCAAGCGATATCAAGGCGATTAACGAGTTCGTTAGTAGGGTAGAGGCAGGAGTTATAAAGG
TTAATAAGCCAACAGTCGGACTGGAATTGCAAGCACCATTTGGTGGTTTTAAGAATTCTGGAGCCACTA
CGTGGAAAGAGATGGGAGAAGATGCTTTAGAGTTCTACCTTAAGGAGAAGACAGTATACGAAGGCTG
GTAA
SEQ ID Nr. 8:

MKSYQGLADKWIKGSGEEYLDINPADKDHVLAKIRLYTKDDVKEAINKAVAKFDEWSRTPAPKRGSILLKAG
ELMEQEAQEFALLMTLEEGKTLKDSMFEVTRSYNLLKFYGALAFKISGKTLPSADPNTRIFTVKEPLGVVALIT
PWNFPLSIPVWKLAPALAAGNTAVIKPATKTPLMVAKLVEVLSKAGLPEGVVNLVVGKGSEVGDTIVSDDNI
AAVSFTGSTEVGKRIYKLVGNKNRMTRIQLELGGKNALYVDKSADLTLAAELAVRGGFGLTGQSCTATSRLII
NKDVYTQFKQRLLERVKKWRVGPGTEDVDMGPVVDEGQFKKDLEYIEYGKNVGAKLIYGGNIIPGKGYFLE
PTIFEGVTSDMRLFKEEIFGPVLSVTEAKDLDEAIRLVNAVDYGHTAGIVASDIKAINEFVSRVEAGVIKVNKPT
VGLELQAPFGGFKNSGATTWKEMGEDALEFYLKEKTVYEGW

Die hier angeführten Aldehyd-Dehydrogenasen umfassen eine Aminosäuresequenz, die eine Identität zu SEQ ID Nr. 2, SEQ ID Nr. 4, SEQ ID Nr. 6 oder SEQ ID Nr. 8 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist.

Alternativ umfassen die Aldehyd-Dehydrogenasen eine Aminosäuresequenz, die durch eine Nukleinsäure kodiert wird, die eine Identität zu SEQ ID Nr. 1, SEQ ID Nr. 3, SEQ ID Nr. 5 oder SEQ ID Nr. 7 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die Aldehyd-Dehydrogenase kodiert, die Nukleinsäuresequenz SEQ ID Nr. 1, SEQ ID Nr. 3, SEQ ID Nr. 5 oder SEQ ID Nr. 7 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990) verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 11, eine Erwartung (E) von 10, M = 5, N = -4 und einen Vergleich beider Stränge. Für Aminosäuresequenzen verwendet das blastp-Programm als Vorgaben eine Wortlänge von 3 und eine Erwartung (E) von 10 und die BLOSUM62-Scoring-Matrix (Henikoff & Henikoff, 1989), Alignments (B) von 50, Erwartung (E) von 10, M = 5, N = -4.

Alternativ umfassen die Aldehyd-Dehydrogenasen eine Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 1, SEQ ID Nr. 3, SEQ ID Nr. 5 oder SEQ ID Nr. 7 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70 °C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Die Erfindung betrifft auch die Verwendung einer Oxidoreduktase, vorzugsweise einer Aldehyd-Dehydrogenase, zur Oxidation eines 5-Formyl-2-furancarbonsäurealkylesters, vorzugsweise der allgemeinen Formel (II) 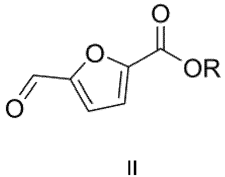 in der R für C1-C4-Alkyl steht, zu einem 2,5-Furandicarbonsäurealkylester, vorzugsweise der allgemeinen Formel (I) 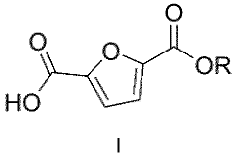 in der R für C1-C4-Alkyl steht, wobei die Oxidoreduktase vorzugsweise eine Aldehyd-Dehydrogenase ist, welche vorzugsweise eine Aminosäuresequenz umfasst oder daraus besteht,, welche ausgewählt ist aus der Gruppe bestehend aus:
i) einer Aminosäuresequenz, die eine Identität zu SEQ ID Nr. 2, SEQ ID Nr. 4, SEQ ID Nr. 6 oder SEQ ID Nr. 8 von mindestens 80% aufweist,
ii) einer Aminosäuresequenz, die durch eine Nukleinsäure kodiert wird, die eine Identität zu SEQ ID Nr. 1, SEQ ID Nr. 3, SEQ ID Nr. 5 oder SEQ ID Nr. 7 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 1, SEQ ID Nr. 3, SEQ ID Nr. 5 oder SEQ ID Nr. 7 bindet.

Besonders bevorzugt umfasst die NAD(P)H-Oxidase eine Aminosäuresequenz oder besteht aus dieser, welche ausgewählt ist aus der Gruppe bestehend aus:
i) einer Aminosäuresequenz, die eine Identität zu SEQ ID Nr. 10 von mindestens 80% aufweist,
ii) einer Aminosäuresequenz, die durch eine Nukleinsäure kodiert wird, die eine Identität zu SEQ ID Nr. 9 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 9 bindet.

SEQ ID Nr. 9: 
ATGAAAGTAGTAGTAGTAGGCTGTACACATGCAGGAACAGCGGCAGTTAAGACGATTTTAAATGAACA
TCCAGATGCATCAGTATCAGTATATGAGCGTAATGACAATGTCTCATTTCTATCTTGTGGGATTGCGTTG
TATGTTGGTGGAGTTGTGAAAGATCCTGCAGGTTTGTTTTATTCAAGTCCAGAAGAACTTGCATCAATG
GGCGCGAAAATTAACATGGAACACAATGTGAAAAATATAGATAATGAGAATAAGGTCGTAGTAATTGA
GAATTTAAAAACAGGCGAAACATTTGAAGAAAGCTATGATAAGTTGGTAATGACAACTGGATCATGGC
CAATTATTCCTCCAATTGATGGAATCAATAGTGAAAATATTCTTTTGTGTAAAAACTATAACCAAGCAAA
TGAAATTATTAAAGAATCAAAAAATGCTAAAAAGATTGTCATTGTTGGTGGTGGCTATATTGGAATTGA
ATTAGTTGAGGCATTTGCAGAATCTGGCAAGCAAGTGACGCTAGTTGATGGATTAGATCGTATTTTAAA
CAAATATTTAGATGCTGAATTCACTTCTGTTTTAGAGCATGATTTACAAGAAAGAGGCGTTACGCTAGCT
TTAAACCAAACCGTCGAGAAATTTGTTGCCAATGAATCAGGTGCTGTGACAGCTGTGAAAACACCAGTT
GGAGAATATGAGGCTGATTTAGTTATTTATGTGTTGGATTTAAACCAAATACTGATTTGTTGAAGGAT
AAAGTAGAGATGTTGCCAAATGGTGCCATCGTAGTGGATGAATATATGAGAACAAGCGATGAAGCGAT
TTTTGCTGCTGGCGATAGTTGCGCGGTTCATTATAATCCAACTGGAGGCTCTGCGTATATTCCGTTAGCT
ACAAATGCAGTTAGAATGGGAGCTTTAGTTGGGAAAAATATTGTTTCTCCAACAGTTAAATATCGTGGC
ACGCAAGCAACTTCTGGTTTATATTTATTTGGTTTTAATATAGGTTCAACCGGATTGACTGAAAATAGCG
CTCCTCATTTTGGCGTAGAGGTTCGTTCAGTAGTTGTAGAAGATAATTATCGTCCAGAGTTTATGCCGAC
AACAGAGAAAGTAACGATGAAATTAGTTTATGAAGTAGGAACGAATCGGATTGTTGGAGGTCAAATCA
TGTCAAAATATGATGTGACACAATCTGCCAATACGTTATCTTTATGTGTTCAAAATAAAATGACGATTGA
GGATTTGGCTTATGTAGATTTCTTCTTCCAACCTCACTTTGATCGTCCTTGGAACTATTTAAATATTTTAG
CGCAAGCAGCTGTTGAGCAAGAGCGTAAACTAGCAAAATAA
SEQ ID Nr. 10:

MKVVVVGCTHAGTAAVKTILNEHPDASVSVYERNDNVSFLSCGIALYVGGVVKDPAGLFYSSPEELASMGAKIN
MEHNVKNIDNENKVVVIENLKTGETFEESYDKLVMTTGSWPIIPPIDGINSENILLCKNYNQANEIIKESKNAKKIV
IVGGGYIGIELVEAFAESGKQVTLVDGLDRILNKYLDAEFTSVLEHDLQERGVTLALNQTVEKFVANESGAVTAVK
TPVGEYEADLVILCVGFKPNTDLLKDKVEMLPNGAIVVDEYMRTSDEAIFAAGDSCAVHYNPTGGSAYIPLATN
AVRMGALVGKNIVSPTVKYRGTQATSGLYLFGFNIGSTGLTENSAPHFGVEVRSVVVEDNYRPEFMPTTEKVT
MKLVYEVGTNRIVGGQIMSKYDVTQSANTLSLCVQNKMTIEDLAYVDFFFQPHFDRPWNYLNILAQAAVEQER
KLAK

Die NAD(P)H-Oxidase umfasst oder besteht vorzugsweise aus einer Aminosäuresequenz, die eine Identität zu SEQ ID Nr. 10 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die NAD(P)H-Oxidase die Aminosäuresequenz SEQ ID Nr. 10 oder besteht aus dieser.

Alternativ umfasst die NAD(P)H-Oxidase vorzugsweise eine Aminosäuresequenz, die durch eine Nukleinsäure kodiert wird, die eine Identität zu SEQ ID Nr. 9 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die NAD(P)H-Oxidase kodiert, die Nukleinsäuresequenz SEQ ID Nr. 9 oder besteht aus dieser.

Alternativ umfasst die NAD(P)H-Oxidase eine Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 9 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70 °C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Ein weiterer Aspekt der vorliegenden Erfindung betrifft die Verwendung einer NAD(P)H-Oxidase zur Oxidation von NADH zu NAD⁺ und/oder NADPH zu NADP⁺, vorzugsweise bei der Herstellung eines 2,5-Furandicarbonsäurealkylesters, vorzugsweise der allgemeinen Formel (I), in der R für C1-C4-Alkyl steht, aus einem 5-Formyl-2-furancarbonsäurealkylester, vorzugsweise der allgemeinen Formel (II), in der R für C1-C4-Alkyl steht, welche eine Aminosäuresequenz umfasst oder daraus besteht, die ausgewählt ist aus der Gruppe bestehend aus:
i) einer Aminosäuresequenz, die eine Identität zu SEQ ID Nr. 10 von mindestens 80% aufweist,
ii) einer Aminosäuresequenz, die durch eine Nukleinsäure kodiert wird, die eine Identität zu SEQ ID Nr. 9 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 9 bindet.

Die erfindungsgemäße NAD(P)H-Oxidase kann in einem Cofaktor-Recyclingsystem oder als Cofaktor-Recyclingsystem verwendet werden, in dem NADH zu NAD⁺ und/oder NADPH zu NADP⁺ oxidiert wird. Cofaktor-Recyclingsysteme werden vorzugsweise in enzymatischen Reaktionen eingesetzt, in denen NADH und/oder NADPH aus NAD⁺ bzw. NADP⁺ gebildet wird.

Besonders bevorzugt umfasst die Alkoholdehydrogenase eine Aminosäuresequenz oder besteht aus dieser, welche ausgewählt ist aus der Gruppe bestehend aus:
i) einer Aminosäuresequenz, die eine Identität zu SEQ ID Nr. 12 von mindestens 80% aufweist,
ii) einer Aminosäuresequenz, die durch eine Nukleinsäure kodiert wird, die eine Identität zu SEQ ID Nr. 11 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 11 bindet.

SEQ ID Nr. 11: 
ATGAAAGGTTTTGCAATGCTCAGTATCGGTAAAGTTGGCTGGATTGAGAAGGAAAAGCCTGCTCCTGGC
CCATTTGATGCTATTGTAAGACCTCTAGCTGTGGCCCCTTGCACTTCGGACATTCATACCGTTTTTGAAGG
CGCCATTGGCGAAAGACATAACATGATACTCGGTCACGAAGCTGTAGGTGAAGTAGTTGAAGTAGGTAG
TGAGGTAAAAGATTTTAAACCTGGTGATCGCGTTGTTGTGCCAGCTATTACCCCTGATTGGCGGACCTCT
GAAGTACAAAGAGGATATCACCAGCACTCCGGTGGAATGCTGGCAGGCTGGAAATTTTCGAATGTAAAA
GATGGTGTTTTTGGTGAATTTTTTCATGTGAATGATGCTGATATGAATTTAGCACATCTGCCTAAAGAAAT
TCCATTGGAAGCTGCAGTTATGATTCCCGATATGATGACCACTGGTTTTCACGGAGCTGAACTGGCAGAT
ATAGAATTAGGTGCGACGGTAGCAGTTTTGGGTATTGGCCCAGTAGGTCTTATGGCAGTCGCTGGTGCC
AAATTGCGTGGAGCCGGAAGAATTATTGCCGTAGGCAGTAGACCAGTTTGTGTAGATGCTGCAAAATAC
TATGGAGCTACTGATATTGTAAACTATAAAGATGGTCCTATCGAAAGTCAGATTATGAATCTAACTGAAG
GCAAAGGTGTCGATGCTGCCATCATCGCTGGAGGAAATGCTGACATTATGGCTACAGCAGTTAAGATTG
TTAAACCTGGTGGCACCATCGCTAATGTAAATTATTTTGGCGAAGGAGAGGTTTTGCCTGTTCCTCGTCTT
GAATGGGGTTGCGGCATGGCTCATAAAACTATAAAAGGCGGGCTATGCCCCGGTGGACGTCTAAGAATG
GAAAGACTGATTGACCTTGTTTTTTATAAGCGTGTCGATCCTTCTAAGCTCGTCACTCACGTTTTCCGGGG
ATTTGACAATATTGAAAAAGCCTTTATGTTGATGAAAGACAAACCAAAAGACCTAATCAAACCTGTTGTA
ATATTAGCATAA
SEQ ID Nr. 12:

MKGFAMLSIGKVGWIEKEKPAPGPFDAIVRPLAVAPCTSDIHTVFEGAIGERHNMILGHEAVGEVVEVGSEVK
DFKPGDRVVVPAITPDWRTSEVQRGYHQHSGGMLAGWKFSNVKDGVFGEFFHVNDADMNLAHLPKEIPLE
AAVMIPDMMTTGFHGAELADIELGATVAVLGIGPVGLMAVAGAKLRGAGRIIAVGSRPVCVDAAKYYGATDI
VNYKDGPIESQIMNLTEGKGVDAAIIAGGNADIMATAVKIVKPGGTIANVNYFGEGEVLPVPRLEWGCGMA
HKTIKGGLCPGGRLRMERLIDLVFYKRVDPSKLVTHVFRGFDNIEKAFMLMKDKPKDLIKPVVILA

Die hier angeführte Alkoholdehydrogenase umfasst eine Aminosäuresequenz, die eine Identität zu SEQ ID Nr. 12 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist.

Alternativ umfasst die Alkoholdehydrogenase eine Aminosäuresequenz, die durch eine Nukleinsäure kodiert wird, die eine Identität zu SEQ ID Nr. 11 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die Alkoholdehydrogenase kodiert, die Nukleinsäuresequenz SEQ ID Nr. 11 oder besteht aus dieser.

Alternativ umfasst die Alkoholdehydrogenase eine Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 11 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70 °C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Die Erfindung betrifft auch die Verwendung einer Alkoholdehydrogenase zur Oxidation von NADH zu NAD⁺ und/oder NADPH zu NADP⁺, vorzugsweise bei der Herstellung eines 2,5-Furandicarbonsäurealkylesters, vorzugsweise der allgemeinen Formel (I), in der R für C1-C4-Alkyl steht, aus einem 5-Formyl-2-furancarbonsäurealkylester, vorzugsweise der allgemeinen Formel (II), in der R für C1-C4-Alkyl steht, wobei die Alkoholdehydrogenase eine Aminosäuresequenz umfasst oder daraus besteht, welche ausgewählt ist aus der Gruppe bestehend aus:
i) einer Aminosäuresequenz, die eine Identität zu SEQ ID Nr. 12 von mindestens 80% aufweist,
ii) einer Aminosäuresequenz, die durch eine Nukleinsäure kodiert wird, die eine Identität zu SEQ ID Nr. 11 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 11 bindet.

Die erfindungsgemäße Alkoholdehydrogenase kann in einem Cofaktor-Recyclingsystem oder als Cofaktor-Recyclingsystem verwendet werden, in dem NADH zu NAD⁺ und/oder NADPH zu NADP⁺ oxidiert wird.

Besonders bevorzugt wird das vorliegende Verfahren außerhalb eines Organismus durchgeführt (in *vitro*), das heißt nicht in einer ganzen Zelle und auch nicht fermentativ.

In einer weiteren bevorzugten Ausführungsform des erfindungsgemäßen Verfahrens beträgt die Konzentration der Substrate in der wässerigen Lösung zwischen 5 und 100 g/l.

Der besonders bevorzugte Temperaturbereich der Reaktionen liegt zwischen 20 und 50 °C.

Der besonders bevorzugte pH-Bereich der Reaktionen liegt zwischen pH 5 und 9.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind. Zudem können die Enzyme auch in lyophilisierter oder sprühgetrockneter oder immobilisierter Form im erfindungsgemäßen Verfahren eingesetzt werden. Die erfindungsgemäß eingesetzten Enzyme können auch in einer im Wesentlichen gereinigten Form verwendet werden, so dass die Enzympräparate keine oder im Wesentlichen keine Bestandteile umfassen, die von den Zellen bzw. der Kultur, in der diese exprimiert wurden, stammen. Die beispielsweise durch rekombinante Expression (z.B. in *E. coli*) hergestellten Enzyme können durch Verfahren, die im Stand der Technik bekannt sind, gereinigt werden, um nach u.a. Ultrafiltration und DNA-Entfernung auch in und zur Herstellung von Lebensmitteln eingesetzt zu werden.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

Die mit dem Verfahren erhaltenen 2,5-Furandicarbonsäurealkylester können mit einem geeigneten organischen Lösungsmittel extrahiert werden, womit ionische Verunreinigungen reduziert werden können. Dies ist vorteilhaft für die nachfolgende Polymerisation zu PEF.

In einer weiteren Variante können die 2,5-Furandicarbonsäurealkylester auch durch Zugabe von Säure aus der wässrigen Lösung gefällt werden.

Mit den nachfolgenden Beispielen werden bevorzugte Ausführungsformen der Erfindung noch näher beschrieben.

### Materialien

Triethanolamin, 1-Butanol, Ethylacetat und Natriumdodecylsulfat (SDS) wurden von Carl Roth, 5-Formyl-2-furancarbonsäuremethylester (Methyl-5-formyl-2-furoat) wurde von abcr, Ethanol wurde von Honeywell, IPTG (Isopropyl-β-D-thiogalactopyranosid) und NAD⁺, NADH-Dinatriumsalz, NADP⁺-Dinatriumsalz, NADPH-Tetranatriumsalz, Phosphorsäure wurde von Sigma-Aldrich, 5-Formyl-2-furancarbonsäure (FFA) wurde von TCI, sowie Acetonitril und Aceton wurden von PanReac AppliChem (ITW Reagents) bezogen.

5-Formyl-2-furancarbonsäureethylester (Ethyl-5-formyl-2-furoat) und 5-Formyl-2-furancarbonsäure-n-butylester (n-Butyl-5-formyl-2-furoat) wurden durch säurekatalysierte Veresterung (Fischer-Veresterung) von 5-Formyl-2-furancarbonsäure mit den entsprechenden Alkoholen hergestellt.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E. coli*-Zellen Top10F' transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optischen Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymtypen und Spenderorganismen für die in den Beispielen verwendeten Enzyme.**

| **Enzymtyp** | **katalysierte Reaktion** | **Spenderorganismus** | **Sequenzen/Literatur** |
|---|---|---|---|
| Aldehyd-Dehydrogenase I | 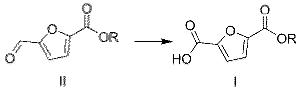 | *Pseudomonas nitroreducens* | (NCBI Protein Database: WP_024766379.1); SEQ ID Nr. 2 |
| Aldehyd-Dehydrogenase II | | *Methylovorus glucosotrophus* | (NCBI Protein Database: WP_015829138.1); SEQ ID Nr. 4 |
| Aldehyd-Dehydrogenase III | | *Pseudomonas aeruginosa* | (UniProt Database: Q9HUR4); SEQ ID Nr. 6 |
| Aldehyd-Dehydrogenase IV* | | *Saccharolobus solfataricus* | (NCBI Protein Database: WP_009990943.1); SEQ ID Nr. 8 |
| NAD(P)H-Oxidase | NAD(P)H → NAD(P)⁺ | *Carnobacterium divergens* | SEQ ID Nr. 10 |
| Alkoholdehydrogenase | Aceton → 2-Propanol | *Thermoanaerobacter brockii* | (NCBI Protein Database: CAA46053.1); SEQ ID Nr. 12 |

| | | | |
|---|---|---|---|
| *Diese ALDH wird in der NCBI Protein Database (Eintrag WP_009990943.1) als 2,5-Dioxopentanoat-Dehydrogenase (katalysiert die Oxidation von 2,5-Dioxopentanoat zu α-Ketoglutarat) klassifiziert. | | | |

### Analytische Methoden

### High Performance Liquid Chromatography (HPLC)

Zur Quantifizierung der Analyten mittels HPLC (High Performance Liquid Chromatography) wurde ein Agilent HPLC 1260 Infinity II Series System verwendet.

Zur Messung mit UV-Detektion (245 nm) wurde eine Merck Purosphere^{®} STAR RP-18 endcapped (5µm) Säule mit entsprechender Vorsäule verwendet, wobei die Elution mit einer Gradientenmethode aus wässriger Phosphorsäure (pH 2,6) und Acetonitril erfolgte.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung oder der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67.10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Oxidation von 5-Formyl-2-furancarbonsäuremonomethylester (FFA-Methylester) zu 2,5-Furandicarbonsäuremonomethylester (FDCA-Monomethylester)

In zwei Vials (2 ml) wurden folgende Komponenten vermischt: 5 mg FFA-Methylester, 125 µl eines 1 M Kaliumphosphat-Puffers (pH 7), 10 µl Aldehyd-Dehydrogenase-Lysat (siehe nachfolgende Tabelle 2), 5 µl einer 5 mM NADP⁺-Lösung (finale Konzentration 0,05 mM) oder 15 µl einer 10 mM NAD⁺-Lösung (finale Konzentration 0,3 mM) sowie 10 U NAD(P)H-Oxidase (NOX)-Lysat oder 10 U Alkoholdehydrogenase-Lysat (ADH; siehe nachfolgende Tabelle 2). Beide Ansätze wurden mit deionisiertem Wasser auf 500 µl Gesamtvolumen aufgefüllt. Zusätzlich enthielt jener Ansatz mit Alkoholdehydrogenase 15 µl Aceton.

Die Ansätze wurden 20 h bei 30 °C unter kontinuierlichem Schütteln (Eppendorf Thermomixer) inkubiert.

Zur Aufarbeitung wurden 50 µl eines Ansatzes mit 200 µl Acetonitril versetzt und bei 85 °C für 15 min inkubiert (Eppendorf Thermomixer). Nach der Denaturierung wurde die Probe mit 750 µl Reinstwasser verdünnt und im Anschluss 10 min bei max. g zentrifugiert. Der Überstand wurde 1:1 mit 20 v% Acetonitril in Wasser verdünnt, in ein HPLC-Vial überführt und vermessen (HPLC, UV-Detektion).

In beiden Ansätzen wurde der FFA-Methylester komplett zum FDCA-Monomethylester oxidiert.

**Tabelle 2.**

| **Vial** | **Aldehyd-Dehydrogenase** | **Regeneration** | **NAD/NADP** |
|---|---|---|---|
| 1 | ALDH I | NAD(P)H-Oxidase | NAD |
| 2 | ALDH III | Alkoholdehydrogenase | NADP |

### Beispiel 2

### Oxidation von 5-Formyl-2-furancarbonsäuremonoethylester (FFA-Ethylester) oder 5-Formyl-2-furancarbonsäuremono-n-butylester (FFA-n-Butylester) zu 2,5-Furandicarbonsäuremonoethylester (FDCA-Monoethylester) oder 2,5-Furandicarbonsäuremono-n-butylester (FDCA-Mono-n-butylester)

In vier Vials (2 ml) wurden folgende Komponenten vermischt: 12,5 µl FFA-Ethylester oder FFA-n-Butylester gelöst in Aceton (entspricht 5 mg Substrat), 125 µl eines 1 M Kaliumphosphat-Puffers (pH 7), 10 µl Aldehyd-Dehydrogenase-Lysat (ALDH, siehe nachfolgende Tabelle 3), 5 µl einer 5 mM NADP⁺-Lösung (finale Konzentration 0,05 mM) oder 15 µl einer 10 mM NAD⁺-Lösung (finale Konzentration 0,3 mM) sowie 10 U NAD(P)H-Oxidase-Lysat oder 10 U Alkoholdehydrogenase-Lysat (siehe nachfolgende Tabelle 3). Alle Ansätze wurden mit deionisiertem Wasser auf 500 µl Gesamtvolumen aufgefüllt. Zusätzlich enthielten jene Ansätze mit Alkoholdehydrogenase 15 µl Aceton.

Die Ansätze wurden 20 h bei 30 °C unter kontinuierlichem Schütteln (Eppendorf Thermomixer) inkubiert.

Zur Aufarbeitung wurden 50 µl eines Ansatzes mit 200 µl Acetonitril versetzt und bei 85 °C für 15 min inkubiert (Eppendorf Thermomixer). Nach der Denaturierung wurde die Probe mit 750 µl Reinstwasser verdünnt und im Anschluss 10 min bei max. g zentrifugiert. Der Überstand wurde in ein HPLC-Vial überführt und vermessen (HPLC, UV-Detektion).

In allen Ansätzen wurden die eingesetzten FFA-Ester komplett zu den entsprechenden FDCA-Monoestern oxidiert.

**Tabelle 3.**

| **Vial** | | **Aldehyd-Dehydrogenase** | **Regeneration** | **NAD/NADP** |
|---|---|---|---|---|
| 1 | FFA-Ethylester | ALDH I | NAD(P)H-Oxidase | NAD |
| 2 | | ALDH III | Alkoholdehydrogenase | NADP |
| 3 | FFA-n-Butylester | ALDH I | NAD(P)H-Oxidase | NAD |
| 4 | | ALDH III | Alkoholdehydrogenase | NADP |

### Beispiel 3

### Oxidation von 5-Formyl-2-furancarbonsäuremonomethylester (FFA-Methylester) zu 2,5-Furandicarbonsäuremonomethylester (FDCA-Monomethylester) mit anschließender Produktisolation

In einem 100 ml Rundkolben wurden 500 mg FFA-Methylester mit einem Magnetrührstäbchen vorgelegt und mit 33 ml deionisiertem Wasser, 12,5 ml eines 1 M Kaliumphosphat-Puffers (pH 7), 1,5 ml Aceton, 0,5 ml einer 5 mM NADP⁺-Lösung, 1 ml Aldehyd-Dehydrogenase III-Lysat sowie 1 ml Alkoholdehydrogenase-Lysat versetzt und bei 30 °C (Heizbad) unter Rühren (350 rpm) inkubiert.

Während der Reaktion wurden Verlaufsproben genommen und analysiert. Dazu wurden 50 µl des Ansatzes mit 200 µl Acetonitril versetzt und bei 85 °C für 15 min inkubiert (Eppendorf Thermomixer). Nach der Denaturierung wurde die Probe mit 750 µl Reinstwasser verdünnt und im Anschluss 10 min bei max. g zentrifugiert. Der Überstand wurde in ein HPLC-Vial überführt und vermessen (HPLC, UV-Detektion).

Nach 18 h konnte ein vollständiger Umsatz festgestellt werden.

Zur Aufarbeitung wurde der pH-Wert der Reaktionsmischung zuerst mit 1 ml 1 M H₂SO₄ auf 3 gestellt. Danach wurde die Mischung dreimal mit je 50 ml Ethylacetat extrahiert. Die organischen Phasen wurden vereint, über Na₂SO₄ getrocknet und das Lösungsmittel wurde am Rotationsverdampfer durch Anlegen eines Unterdruckes entfernt. Der erhaltene Feststoff wurde im Vakuumtrockenschrank für 48 h getrocknet.

Auf diese Weise konnte FDCA-Monomethylester als gelblicher Feststoff (327 mg;) gewonnen werden.

### Literatur

Carus, M., Dammer, L., Raschka, A., Skoczinski, P., & vom Berg, C. (2020). Renewable Carbon-Key to a Sustainable and Future-Oriented Chemical and Plastic Industry. https://renewable-carbon-initiative.com/wp-content/uploads/2020/09/20-09-21_Paper_12-on-Renewable-Carbon.pdf
Hamilton, L. A., Feit, S., Muffett, C., Kelso, M., Rubright, S. M., Bernhardt, C., Schaeffer, E., Moon, D., Morris, J., & Labbé-Bellas, R. (2019). Plastic & Climate: The Hidden Costs of a Plastic Planet. https://www.ciel.org/wp-content/uploads/2019/05/Plastic-and-Climate-FINAL-2019.pdf
Tomás, R. A. F., Bordado, J. C. M., & Gomes, J. F. P. (2013). p-Xylene Oxidation to Terephthalic Acid: A Literature Review Oriented toward Process Optimization and Development. Chemical Reviews, 113(10), 7421-7469. https://doi.org/10.1021/cr300298j
Berger, A. (2016). Ethylenglycol, RD-05-01999 in Böckler, F., Dill, B., Eisenbrand, G., Faupel, F., Fugmann, B., Gamse, T., Matissek, R., Pohnert, G., Rühling, A., Schmidt, S., Sprenger, G. (Ed.), RÖMPP [Online], Stuttgart, Georg Thieme Verlag. https://roempp.thieme.de/lexicon/RD-05-01999
Fan, D., Dai, D.-J., Wu, H.-S. (2013). Ethylene Formation by Catalytic Dehydration of Ethanol with Industrial Considerations. Materials 6(1), 101-115. https://doi.org/10.3390/ma6010101
Salusjärvi, L., Havukainen, S., Koivistoinen, O., & Toivari, M. (2019). Biotechnological production of glycolic acid and ethylene glycol: current state and perspectives. Applied Microbiology and Biotechnology, 103, 2525-2535. https://doi.org/10.1007/s00253-019-09640-2
de Jong, E., Visser, H. A., Dias, A. S., Harvey, C., Gruter, G.-J. M. (2022). The Road to Bring FDCA and PEF to the Market. Polymers, 14, 943. https://doi.org/10.3390/polym14050943
Yu, Z., Zhou, J., Cao, F., Zhang, Q., Huang, K., & Wei, P. (2016). Synthesis, Characterization and Thermal Properties of Bio-Based Poly(Ethylene 2,5-Furan Dicarboxylate). Journal of Macromolecular Science, Part B: Physics, 55(12), 1135-1145. https://doi.org/10.1080/00222348.2016.1238335
Salazar, A., Linke, A., Eckelt, R., Quade, A., Kragl, U., & Mejía, E. (2020). Oxidative Esterification of 5-Hydroxymethylfurfural under Flow Conditions Using a Bimetallic Co/Ru Catalyst. ChemCatChem, 12(13), 3504-3511. https://doi.org/10.1002/cctc.202000205
Chen, J., Cai, J., Sha, F., Sun, W., Lyu, X., Chang, Y., Cao, F., Zhao, L., Wu, H., & Ouyang, P. (2023). Chemoenzymatic cascades producing 2,5-furandicarboxylic acid precursors via D-gluconate "barbell oxidation" and dehydration. Green Chemistry, 25(18), 7126-7140. https://doi.org/10.1039/D3GC01493B
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Protein [Internet]. Bethesda (MD): National Library of Medicine (US), National Center for Biotechnology Information. Accession No. WP_024766379.1, aldehyde dehydrogenase family protein [Pseudomonas nitroreducens]. Verfügbar unter: https://www.ncbi.nlm.nih.gov/protein/WP_024766379.1/
Protein [Internet]. Bethesda (MD): National Library of Medicine (US), National Center for Biotechnology Information. Accession No. WP_015829138.1, aldehyde dehydrogenase family protein [Methylovorus glucosotrophus]. Verfügbar unter: https://www.ncbi.nlm.nih.gov/protein/WP_015829138.1/
UniProt [Internet]. Probable aldehyde dehydrogenase [Pseudomonas aeruginosa]. Entry: Q9HUR4 · Q9HUR4_PSEAE. https://www.uniprot.org/uniprotkb/Q9HUR4/entry
Protein [Internet]. Bethesda (MD): National Library of Medicine (US), National Center for Biotechnology Information. Accession No. WP_009990943.1, 2,5-dioxopentanoate dehydrogenase [Saccharolobus solfataricus]. Verfügbar unter: https://www.ncbi.nlm.nih.gov/protein/WP_009990943.1/
Protein [Internet]. Bethesda (MD): National Library of Medicine (US), National Center for Biotechnology Information. Accession No. CAA46053.1, alcohol dehydrogenase [Thermoanaerobacter brockii]. Verfügbar unter: https://www.ncbi.nlm.nih.gov/protein/CAA46053.1

## Patentansprüche

1. Verfahren zur Herstellung eines 2,5-Furandicarbonsäurealkylesters der allgemeinen Formel (I) 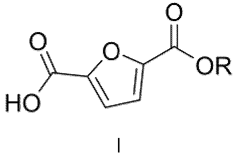
in der R für C1-C4-Alkyl steht,
indem ein 5-Formyl-2-furancarbonsäurealkylester der allgemeinen Formel (II) 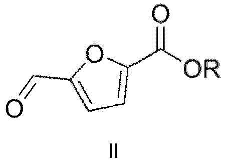
in der R für C1-C4-Alkyl steht,
und welcher in einer wässrigen Lösung vorliegt, durch Behandeln mit einer ersten NAD(P)⁺-abhängigen Oxidoreduktase oxidiert wird, wobei das bei der Oxidation entstandene NAD(P)H enzymatisch mittels einer zweiten Oxidoreduktase wieder zu NAD(P)⁺ oxidiert wird, wonach der 2,5-Furandicarbonsäurealkylester der allgemeinen Formel (I) abgetrennt wird.

2. Verfahren nach Anspruch 1, **dadurch gekennzeichnet, dass** der 5-Formyl-2-furancarbonsäurealkylester ein Methylester (R = CH₃) ist.

3. Verfahren nach einem der Ansprüche 1 oder 2, **dadurch gekennzeichnet, dass** die erste Oxidoreduktase eine Aldeyhd-Dehydrogenase ist.

4. Verfahren nach Anspruch 3, **dadurch gekennzeichnet, dass** die Aldehyd-Dehydrogenase eine Aminosäuresequenz umfasst, welche ausgewählt ist aus der Gruppe bestehend aus:
i) einer Aminosäuresequenz, die eine Identität zu SEQ ID Nr. 2, SEQ ID Nr. 4, SEQ ID Nr. 6 oder SEQ ID Nr. 8 von mindestens 80% aufweist,
ii) einer Aminosäuresequenz, die durch eine Nukleinsäure kodiert wird, die eine Identität zu SEQ ID Nr. 1, SEQ ID Nr. 3, SEQ ID Nr. 5 oder SEQ ID Nr. 7 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 1, SEQ ID Nr. 3, SEQ ID Nr. 5 oder SEQ ID Nr. 7 bindet.

5. Verfahren nach einem der Ansprüche 1 bis 4, **dadurch gekennzeichnet, dass** die zweite Oxidoreduktase eine Dehydrogenase oder eine NAD(P)H-Oxidase ist.

6. Verfahren nach Anspruch 5, **dadurch gekennzeichnet, dass** die Dehydrogenase eine Alkoholdehydrogenase ist, die vorzugsweise eine Aminosäuresequenz umfasst, welche ausgewählt ist aus der Gruppe bestehend aus:
i) einer Aminosäuresequenz, die eine Identität zu SEQ ID Nr. 12 von mindestens 80% aufweist,
ii) einer Aminosäuresequenz, die durch eine Nukleinsäure kodiert wird, die eine Identität zu SEQ ID Nr. 11 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 11 bindet.

7. Verfahren nach Anspruch 5, **dadurch gekennzeichnet, dass** die NAD(P)H-Oxidase eine Aminosäuresequenz umfasst, welche ausgewählt ist aus der Gruppe bestehend aus:
i) einer Aminosäuresequenz, die eine Identität zu SEQ ID Nr. 10 von mindestens 80% aufweist,
ii) einer Aminosäuresequenz, die durch eine Nukleinsäure kodiert wird, die eine Identität zu SEQ ID Nr. 9 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 9 bindet.

8. Verfahren nach einem der Ansprüche 1 bis 7, **dadurch gekennzeichnet, dass** das Verfahren in *vitro* durchgeführt wird.

9. Verwendung einer Oxidoreduktase, vorzugsweise einer Aldehyd-Dehydrogenase, zur Oxidation eines 5-Formyl-2-furancarbonsäurealkylesters, vorzugsweise der allgemeinen Formel (II) 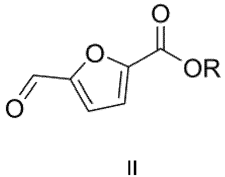 in der R für C1-C4-Alkyl steht, zu einem 2,5-Furandicarbonsäurealkylester, vorzugsweise der allgemeinen Formel (I) 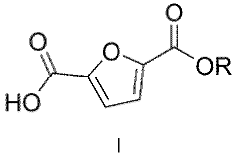 in der R für C1-C4-Alkyl steht, wobei die Oxidoreduktase vorzugsweise eine Aldehyd-Dehydrogenase ist, welche vorzugsweise eine Aminosäuresequenz umfasst, welche ausgewählt ist aus der Gruppe bestehend aus:
i) einer Aminosäuresequenz, die eine Identität zu SEQ ID Nr. 2, SEQ ID Nr. 4, SEQ ID Nr. 6 oder SEQ ID Nr. 8 von mindestens 80% aufweist,
ii) einer Aminosäuresequenz, die durch eine Nukleinsäure kodiert wird, die eine Identität zu SEQ ID Nr. 1, SEQ ID Nr. 3, SEQ ID Nr. 5 oder SEQ ID Nr. 7 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 1, SEQ ID Nr. 3, SEQ ID Nr. 5 oder SEQ ID Nr. 7 bindet.

10. Verwendung einer NAD(P)H-Oxidase zur Oxidation von NADH zu NAD⁺ und/oder NADPH zu NADP⁺, vorzugsweise bei der Herstellung eines 2,5-Furandicarbonsäurealkylesters, vorzugsweise der allgemeinen Formel (I), in der R für C1-C4-Alkyl steht, aus einem 5-Formyl-2-furancarbonsäurealkylester, vorzugsweise der allgemeinen Formel (II), in der R für C1-C4-Alkyl steht, welche eine Aminosäuresequenz umfasst oder daraus besteht, die ausgewählt ist aus der Gruppe bestehend aus:
i) einer Aminosäuresequenz, die eine Identität zu SEQ ID Nr. 10 von mindestens 80% aufweist,
ii) einer Aminosäuresequenz, die durch eine Nukleinsäure kodiert wird, die eine Identität zu SEQ ID Nr. 9 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 9 bindet.

11. Verwendung einer Alkoholdehydrogenase zur Oxidation von NADH zu NAD⁺ und/oder NADPH zu NADP⁺, vorzugsweise bei der Herstellung eines 2,5-Furandicarbonsäurealkylesters, vorzugsweise der allgemeinen Formel (I), in der R für C1-C4-Alkyl steht, aus einem 5-Formyl-2-furancarbonsäurealkylester, vorzugsweise der allgemeinen Formel (II), in der R für C1-C4-Alkyl steht, wobei die Alkoholdehydrogenase eine Aminosäuresequenz umfasst oder daraus besteht, welche ausgewählt ist aus der Gruppe bestehend aus:
i) einer Aminosäuresequenz, die eine Identität zu SEQ ID Nr. 12 von mindestens 80% aufweist,
ii) einer Aminosäuresequenz, die durch eine Nukleinsäure kodiert wird, die eine Identität zu SEQ ID Nr. 11 von mindestens 80% aufweist, und
iii) einer Aminosäuresequenz, die durch eine Nukleinsäure kodiert wird, die unter stringenten Bedingungen an einen komplementären Strang eines Nukleinsäuremoleküls mit der Nukleinsäuresequenz SEQ ID Nr. 11 bindet.
